# EUROPEAN PATENT APPLICATION

(11) **EP 1 602 330 A1**
(43) Date of publication of application: **07.12.2005**
(21) Application number: 04447136.5
(22) Date of filing: 04.06.2004
(51) Int. Cl.: A61B 5/22, A61B 5/0488

(54) **Medical device adapted to the monitoring of limb muscle behaviour in patients**

(71) Applicant: UNIVERSITE LIBRE DE BRUXELLES, 1050 Bruxelles (BE); FCS Control Systems B.V., 1117 ZJ Schiphol (NL)
(72) Inventor: Manto, Mario, B-1440 Braine-Le-Château (BE); Van der Linde, Richard, 2662 AN Bergschenhoek (NL); Lammertse, Piet, 1181 SW Amstelveen (NL)
(74) Representative: Van Malderen, Joelle

(57) **Abstract**

The present invention is related to a medical device for monitoring limb muscle behaviour in a patient, said limb corresponding to a leg or an arm, said medical device comprising:
- a moving unit (1) adapted to receive a first part of said leg or arm corresponding to the foot or to the hand (2), said moving unit (1) presenting at least one degree of freedom (11) according to which the moving unit (1) may move;
- a haptic device (6) adapted to receive a second part of said leg or arm corresponding to the foreleg or the forearm (5) respectively, and to measure in the form of an analogical signal the mechanical response of said second part to a movement of the moving unit (1);
- a set of electromyographic electrodes (4) able to measure in the form of an analogical signal the electrical activities in the muscle(s) of said leg or arm respectively;
- an analogical signal acquisition unit (9) able to acquire the analogical signals from the electromyographic electrodes and the haptic device (6) and converting said analogical signals into digital signals;
- a controller (8) able to process said digital signals and to control the moving unit (1).

## Description

### Field of the invention

The present invention is related to a medical device which is preferably portable and a method for monitoring the behaviour of limb muscles, including muscular fibres and motor units, in a patient.

In particular, the present invention is related to a medical device and method for dynamic monitoring of voluntary and involuntary movements in upper or lower limbs of a patient.

### State of the art

Despite recent advances in the management of patients presenting a central nervous system lesion, neurologists and therapists still evaluate voluntary and involuntary movements of upper and lower limbs, and namely movements of the wrist joint, clinically in a subjective manner depending on the examiner. As a consequence, such clinical evaluation lacks reliability.

The interest of a medical device or instrument which could help the examiner in his diagnosis is therefore obvious.

### Aims of the invention

The present invention aims to provide a medical device and method for systematically monitoring limb movements in a patient.

The present invention aims to provide a medical device and method giving reliable data.

The present invention aims to provide a medical device and method easy to use or to implement.

The present invention aims to provide a medical device and method which provide rapidly data interpretable by a trained person.

### Summary of the invention

The present invention is related to a medical device, which is preferably portable, for monitoring limb muscle behaviour in a patient, said limb corresponding to a leg or an arm, said medical device comprising:
- a moving unit adapted to receive a first part of said leg or arm corresponding to the foot or to the hand, said moving unit presenting at least one degree of freedom according to which the moving unit may move;
- a haptic device adapted to receive a second part of said leg or arm corresponding to the foreleg or the forearm respectively, and to measure in the form of an analogical signal the mechanical response of said second part to a movement of the moving unit;
- a set of electromyographic electrodes able to measure in the form of an analogical signal the electrical activities in the muscle(s) of said leg or arm respectively;
- an analogical signal acquisition unit able to acquire the analogical signals from the electromyographic electrodes and the haptic device and converting said analogical signals into digital signals;
- a controller able to process said digital signals and to control the moving unit.

Similarly to the moving unit, the haptic device may also comprise at least one degree of freedom.

In the present description, it is meant by "muscle" the muscle as whole and/or one or more muscular fibres and/or one or more motor units of said muscle.

Preferably, the moving device in operating conditions is driven by a motor under the control of the controller.

Preferably, the electromyographic electrodes comprise needle electrodes and/or surface electrodes and/or microelectrodes. Preferably, the electromyographic electrodes are needle electrodes and/or surface electrodes and/or microelectrodes.

Preferably, the medical device according to invention further comprises an amplification system for amplifying the analogical signals measured by the electrodes.

Preferably, the controller is configured so as to control or maintain in operating conditions in direction and in intensity the driving force of the motor and thereby to control or maintain in direction and in intensity the motion of the moving unit.

Advantageously, the moving unit of the present medical device presents one rotation axis around which the moving unit is able to rotate relatively to the haptic device, preferably with a rotation angle comprised between about +30° and about -30° and a defined maximal rotation velocity.

Preferably, said maximum rotation velocity is about 300°/sec.

Advantageously also, the moving unit has a position accuracy of about 0.1 mm.

The present invention also concerns a method for monitoring the behaviour of a limb muscle using said portable medical device, said limb being either the leg or the arm of a patient. Said method comprises the following steps of :
- placing the foot or the hand respectively of the patient on the moving unit;
- placing the foreleg or the forearm respectively on the haptic device in such a way that the main axis of the ankle or the wrist respectively coincides with the main axis of the motor;
- placing the electromyographic electrodes at different positions in and/or on the muscle of the foreleg or the forearm respectively;
- applying under the control of the controller a force of precise direction and intensity to the motor so as to move with a force of precise direction and intensity the moving unit and the foot or hand respectively placed thereon;
- measuring the mechanical response (direction and intensity of the movements) of the foreleg or forearm respectively by means of the haptic device;
- measuring the electrical response of the foreleg or the forearm by means of the electromyographic electrodes at different locations along said foreleg or forearm respectively;
- acquiring said mechanical and electrical responses in the form of analogical signals by means of the acquiring unit;
- converting said analogical signals into digital signals;
- processing by means of the controller said digital signals.

The method may also comprise the step of applying under the control of the controller a displacement of precise direction and amplitude to the moving unit and the foot or hand respectively placed thereon, and measuring the resulting force at the foreleg or forearm by means of the haptic device.

As already specified hereabove, it is meant by "muscle" the muscle as a whole and/or the muscular fibres and/or the motor units of said muscle.

Preferably, said analogical signals are acquired along time and processed so as to have a monitoring of limb movements along time.

The present invention is also related to the use of the present medical device and/or the present method as a tool for monitoring voluntary and involuntary limb movements in a patient.

The present invention also concerns the use of the present medical device and/or the present method as an indirect tool providing physical data for evaluating the stage of recovery of a patient.

Another object of the present invention is the use of the present medical device and/or the present method for investigating the effects of drugs in a patient.

It has to be noted that the medical device and method according to the present invention provide data corresponding to intermediate results about the limb muscle behaviour which do not on their own enable a decision to be made by the doctor or surgeon on the diagnosis and the treatment necessary.

### Short description of the drawings

Fig. 1 represents an embodiment of a medical device as used for monitoring the behaviour of arm muscles and motor units according to the present invention.

Fig.2a and Fig.2b present respectively a side view and a topview of a medical device (moving unit + haptic device) as used in a preferred embodiment of the present medical device.

### Detailed description of the invention

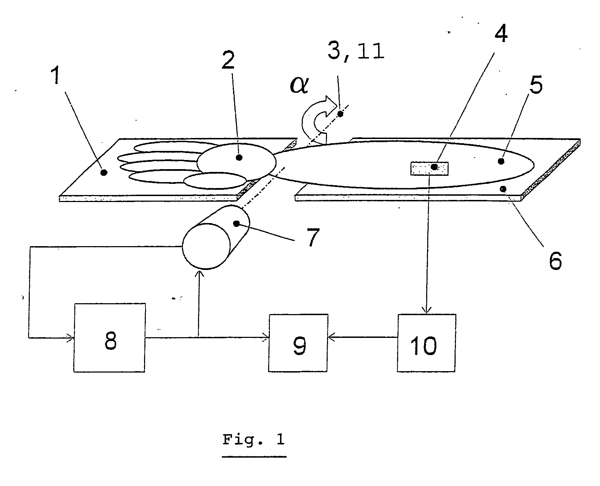

As illustrated in Fig.1, the medical device according to the invention is a portable medical device comprising:
- moving unit 1 able to move according to at least one degree of freedom;
- a motor 7 for driving the moving unit 1, said motor being provided with a main rotation axis 3;
- a haptic device 6 having at least one degree of freedom and being able to measure by tactile contact of an object using haptic technology the mechanical motions, in amplitude and orientation, of said object;
- a set of electromyographic electrodes 4;
- a signal amplification unit 10;
- a signal acquisition unit 9;
- and a controller 8 for driving the motor 7 with calibrated motions and having signal processing capacities.

In the present embodiment, the moving unit presents a rotation axis 11 which coincides with the rotation axis 3 of the motor 7. The moving unit is thus able to rotate around its axis 11 relatively to the haptic device 6, with a precise rotation angle α and at a precise rotation speed or velocity (under the control of the controller 8).

The moving unit 1 could also be able to be translated. Possibly also, the haptic device 6 could be able to be rotated and/or translated.

The haptic device 6 of the present medical device is able to measure forces using the haptic technology.

The principle of said medical device is to induce a mechanical perturbation, preferably via the moving unit 1, in an upper limb or lower limb of a patient and to measure the mechanical and electrical response of the muscles of said upper or lower limb to said perturbations.

The medical device is adapted to the study of a limb muscle as a whole and/of of its muscular fibres and/or of its motor units.

In operating conditions, the method for monitoring the movements of a limb, such as an arm as illustrated in Fig.1, using the present medical device, comprises the following steps:
- the arm is placed relatively to the medical device such that the hand lies on the moving unit 1 and the forearm 5 lies on the haptic device 6, with the main axis of the wrist coinciding with the main axis 3 of the motor 7;
- once the arm is correctly placed, arranging the electromyographic electrodes 4 at different locations along the arm, and preferably along the forearm 5, in the muscles or on the skin of the patient;
- ordering to the motor 7, via the controller 8, to drive the moving unit 1, and thus the hand 2, following a movement with precise direction and amplitude;
- measuring by means of the haptic device 6 the mechanical response in amplitude, orientation, and force of the forearm 5 of the patient;
- simultaneously measuring by means of the electrodes 4 the electrical response (electrical activities) at different locations along the arm, and preferably along the forearm 5;
- acquiring with the acquisition the electrical and mechanical responses in the form of analogical signals;
- converting said analogical signals into digital signals;
- processing by means of the controller 8 said digital signals so as to provide data about the movements of the arm of the patient which could be then interpreted by a trained person.

It should be noted that the medical device may comprise 4 different electromyographic electrodes able to be inserted in four forearm muscles, flexor carpi radialis, extensor carpi radialis, brachioradialis, and flexor carpi ulnaris. Active surface electrodes could be affixed at the skin at the level of the flexor carpi radialis and extensor carpi radialis muscles.

Possibly, the axis of the motor can be moved easily and quickly in more than one plane.

One advantage of the present medical device is that is so configured and dimensioned that it is portable and can be used on the bedside of a patient, with a patient in different possible positions: horizontal, vertical, or seated position. The medical device is thus very comfortable for the patient and could even be used in extreme conditions (emergency situations for example).

The medical device and method according to the invention offer a wide range of applications.

It is possible to identify and analyse the discharges of individual muscular fibres or single motor units in different conditions of impedance of the foreleg or forearm joint.

The present medical device can be combined with a source of external stimuli (preferably electrical or magnetic stimuli), for example over a region of the central nervous system (preferably the motor cortex or the spin cord) or the peripheral nervous system (preferentially a plexus), in order to analyse the control mechanism by the nervous system on the behaviour of motor units and muscular fibres during mechanical stretches of a muscle".

As already disclosed above, the present medical device and method could be used as a tool to investigate the mechanism of neurological control of wrist movements in patients presenting a central nervous system lesion.

For example also, the present medical device and method could be used to analyse a pathological hand tremor through the characterisation of its frequency band and its intermuscular coherence in different impedance conditions an in different conditions of inertia. This analysis could provide indirect information useful in the establishment of a neurological differential diagnosis.

The present medical device can be used as a tool for the clinician which may help him to establish a differential diagnosis of psychogenic tremor.

The present medical device and method could also be used to test the effect of candidate drugs on limb tremor, in particular hand tremor, and for example in essential hand tremor or hand tremor related to Parkinson's disease.

Another possible application of the present medical device and method is their use as a tool to precisely evaluate the mechanism of recovery of cerebellar dysmetria in human and then to determine the stage of recovery reached by a patient following a cerebellar stroke. In that sense, the present medical device and method could indirectly help doctors to improve neurological rehabilitation of patients.

The present medical device and patient could also be used for objectively evaluating the tone of wrist joint during neurosurgery procedures in patients suffering from Parkinson's disease. From that point of view, the present medical device and method could indirectly help doctors to perform a more efficient deep brain stimulation procedure (for example neurostimulation of subthalamic nuclei).

The present medical device can also be used as a tool to analyse hand control in patients after grafting of a hand.

The present medical device could also be used to test prostheses, such as thermoformable prostheses.

Re-education of the limb of a patient after an accident is also a possible application of the present medical device.

As illustrated above, one of the main advantage of the present medical device comparatively to the prior art is to provide objective data.

However, it should be understood that the medical device and method according to the present invention only provide data corresponding to intermediate results about the limb muscle behaviour which do not on their own enable a decision to be made by the doctor or surgeon on the treatment necessary.

### Example of a particular embodiment:

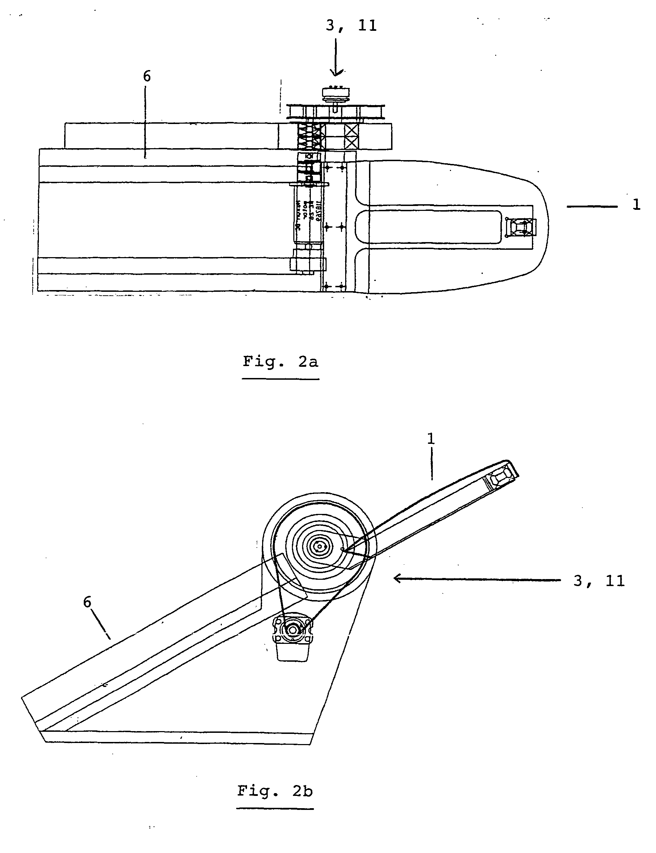

According to a preferred embodiment of the invention, the medical device presents the following technical characteristics:
- 4 differential amplifiers with a gain of 1000 (as for example the ones produced by the Xltek company, Canada);
- 4 sterile concentric needle electrodes 30G (for example as the ones sold by the Medtronic company, (Skovlunde, Denmark));
- two surface active electrodes with preamplification and filters incorporated therein (gain of 1000; filter 20-450 Hz, for example as the ones produced by Delsys DE-2.3);
- 1 analog acquisition unit;
- 1 personal computer; and
- a moving unit coupled to a haptic device as illustrated in Fig.2a and 2b, with one degree of freedom with effort return (rotation axis with numerical reference 11) to generate an arbitrary dynamic behaviour.

The characteristics of said moving unit were the following:
- position accuracy: 0.1 mm at the level of fingers extremities on the haptic device;
- nominal torque: 4 Nm;
- peak torque: 20 Nm;
- maximal rotation velocity: 300 deg/sec
- range of motion: from +30 to -30 degrees.

## Claims

1. A. medical device for monitoring limb muscle behaviour in a patient, said limb corresponding to a leg or an arm, said medical device comprising:
- a moving unit (1) adapted to receive a first part of said leg or arm corresponding to the foot or to the hand (2), said moving unit (1) presenting at least one degree of freedom according to which the moving unit (1) may move;
- a haptic device (6) adapted to receive a second part of said leg or arm corresponding to the foreleg or the forearm (5) respectively, and to measure in the form of an analogical signal the mechanical response of said second part to a movement of the moving unit (1);
- a set of electromyographic electrodes (4) able to measure in the form of an analogical signal the electrical activities in the muscle(s) of said leg or arm respectively;
- an analogical signal acquisition unit (9) able to acquire the analogical signals from the electromyographic electrodes and the haptic device (6) and converting said analogical signals into digital signals;
- a controller (8) able to process said digital signals and to control the moving unit (1).

2. The medical device according to claim 1, **characterized in that** the moving device is driven by a motor (7) under the control of the controller (8).

3. The medical device according to claim 1 or 2, **characterized in that** the electromyographic electrodes are needle electrodes and/or surface electrodes.

4. The medical device according to any one of the preceding claims, **characterized in that** it comprises four electromyographic needle electrode and two electromyographic surface active electrodes.

5. The medical device according to claim 1 or 2, **characterized in that** the electromyographic electrodes are microelectrodes.

6. The medical device according to any one of the preceding claims, **characterized in that** the moving unit (1) presents at least one rotation axis (11) around which the moving unit (1) is able to rotate relatively to the haptic device (6) with a rotation angle (α) comprised between about +30° and about -30° and a defined maximal rotation velocity.

7. The medical device according to claim 6, **characterized in that** the maximum rotation velocity of the moving unit (1) is about 300°/sec.

8. The medical device according to claim 6 or 7, **characterized in that** the moving unit (1) has a position accuracy of about 0.1 mm.

9. The medical device according to any one of the preceding claims, **characterized in that** it further comprises an amplification system for amplifying the analogical signals measured by the electrodes (4).

10. The medical device according to claim 1 or 2, **characterized in that** the controller maintains in direction and in intensity the driving force of the motor (7) and thereby in direction and in intensity the motion of the moving unit (1).
